# EUROPEAN PATENT SPECIFICATION

(11) **EP 1 588 731 B1**
(45) Date of publication and mention of the grant of the patent: **13.06.2007**
(21) Application number: 04076197.5
(22) Date of filing: 20.04.2004
(51) Int. Cl.: A61M 25/00

(54) **Local drug delivery catheter**
Katheter zur lokalen Arzneistoffabgabe
Cathéter d'apport localisé d'un médicament

(43) Date of publication of application: 26.10.2005
(73) Proprietor: Wijay, Bandula, Friendswood, TX 77546 (US); Angelini, Paolo, Houston, TX 77019 (US)
(72) Inventor: Wijay, Bandula, Friendswood, TX 77546 (US); Angelini, Paolo, Houston, TX 77019 (US)
(74) Representative: Elzaburu Marquez, Alberto

(56) References cited:
- WO-A-92/10142
- WO-A-96/35464
- US-A- 5 354 279
- US-A- 5 419 777
- US-B1- 6 302 870
- US-B1- 6 458 098

## Description

The present invention relates to drug injection device for treating tissue in a body cavity with pharmaceutical drugs or other biological techniques (like gene therapy) with the aim of realizing topical treatment as opposed to systemic treatment.

### BACKGROUND OF THE INVENTION

Several medical conditions could potentially be treated more effectively by local administration of therapeutic agents. Recent Studies of the biology of arterial wall have clarified the nature of several localized pathologic changes in the intima, which could be treated effectively with local administration of variety of pharmacologic agents, for example:
- the vulnerable plaque (an atherosclerotic lesion, with high probability of rapid evolution into total occlusion, resulting in dependent tissue death), and
- the restenosis lesions (the fibro cellular proliferative response to the trauma caused by angioplasty interventions onto the vascular wall, leading to recurrent blockage of treated vessel).

Active biological processes in a localizable segment of the circulation can potentially be addressed by local administration of active pharmacologic agents more effectively than by systemic administration route. Both the tissue concentration that can be reached by local delivery and the avoidance of systemic toxicity, besides the diminished dose and cost of the active drug and quickness of its effect, all play in favor of a local administration.

Several alternative methods of local administration of drugs into the desired arterial wall have been developed and tested, namely:
- Endoluminal injection. Catheters have been devised that could obviate to the ineffectiveness of just endo-luminal infusion, while the blood circulation is maintained like temporary double balloon catheter realizing the isolation of the target vascular segment; side-holes injection through especially designed balloons (leaky balloons) or coiled tubing; needle-balloons that are inflated by the same liquid/pressure that is used to inject the active ingredient by the use of communicating needles; and others.

Klein et al. in U.S. Patent 5,810,767 describe one such device where network of tubules attached onto an angioplasty type balloon. The tiny holes in the tubular network would carry the drug to the target region for drug therapy. This type of system obviously blocks the flow of blood distal to the target region and therefore can cause major problems to the patient due to ischemic manifestations.

Hanson et al. in U.S. Patent 5,985,307 also describe a means to deliver drugs by binding the drug to a balloon like device which when inflated or located in the target area will allow the drug to diffuse into the vessel wall, also requiring the stoppage of the flow of fluids in the lumen.

Reed et al. in U.S. Patent 6,97,013 B1, describe a balloon like device containing an array of needles that are affixed to the surface of the balloon. When the balloon is inflated, the needles will puncture into the vessel wall and the pharmacological agents are injected into the wall using the fluid agent to inflate the balloon. These devices are very bulky and are difficult to manipulate into small arteries of the heart. They need to be collapsed when the device is moved within the vessels and the procedure is very cumbersome. And if the device is not collapsed when it is advanced or withdrawn, the device can cause substantial damage to the vessel wall.

Young et al. in U.S. Patent 5,788,673 describe a syringe device for controlling the rate of drug infusion systemically but provides no means to inject the drugs into the vessel wall.

Harrison et al. in U.S. Patent 5,554,119, also describe a balloon like tubular device having tiny holes that would allow the infusion of the pharmacologic agents into the lumen of the vessel. Here again, the drug is easily washed away by the blood stream before it can get diffused into the vessel wall. These devices also tend to be bulky and are quite ineffective in getting the drugs into the luminal wall.

Levy et al. in U.S. Patent 5,833,658 describe another balloon type device where a collar of the balloon allows flooding the pharmacologic or other agents in a vascular segment, where they can diffuse into the vessel wall. Again these types of devices occlude the flow of blood in the lumen during such procedures, which often require long periods for the diffusion of the drugs. As such they are highly ineffective. Schweich et al. in U.S. Patent 5,558,642 describes a similar concept.

Schreiner in U.S. Patent 5,904,670 describes a device that expands once deployed in the vessel due to shape memory characteristics. The device contains needles that can puncture into the vessel wall. While this device allows blood flow during the procedure as compared to other devices that contain a balloon, these wire cages are very cumbersome to use and the orientation of the needles is difficult to maintain, in a biological environment, where often the vessels are tortuous and non linear. Also the push-pull mechanisms that drive the needle assemblies often do not provide sufficient puncture force to puncture the intimal layers and are highly ineffective.

Jacobsen et al. describe a device in their U.S. Patent 6,302,870 that has a group of retractable needles that can be made to project out of a catheter having collars to stop the penetration of the needle. The disadvantages of this design are that often it is impractical to slide a group of tubes with a small catheter tube whose overall diameter is less than 1.5 mm and it is also impractical to be able to stop the penetration of a needle with the use of a tiny needle using a collar whose diameter is only slightly larger than the needle itself Jacobsen et al. also describe an apparatus in which the needles protrude out of a tube by the use of a twisting motion, however in order to obtain sufficient outward movement, the enclosed length has to be fairly large making the tube diameter very large causing the blockage of the lumen and making the device bulky. Additionally, there is no means to control the depth of penetration of the needles into the vessel wall.

Others, such as Haim, U.S. Patent 6,254,573, on the other hand, have developed various means of injecting the drug to the wall of the blood vessel using metallic and non-metallic needles of various sizes and shapes. Although this method does not necessarily block the blood flow to the organ, actually constructing such a device that can accomplish this objective, is not easily done. The metallic "needles" are stiff and difficult to manage and the actual penetration of the needle into the vessel wall cannot be easily controlled. On the other hand "needles" made from plastics and other non-metallic materials often do not have sufficient strength and orientation to penetrate the vessel wall adequately- Any device containing a metallic needle, however small in diameter, tends to make the catheter stiff and not tractable, hence, clinically unusable,

Glines et al. U.S. Patent 6,183,444, have developed "Drug Delivery Module" - type system having a needle attached to a reservoir that is delivered using an endoscope or a catheter. Ahern et al. U.S. Patent 6,251,418 suggests a method of implanting pellets containing the drug in the myocardial tissue.

Still other inventors, such as Ungs, U.S. Patent 6,149,641, have devised other means such as impregnating the drug into a carrier medium or using a porous balloon where by the drug bleeds out of the porous balloon. Winkler et al. U.S. Patent 6,200,257 discuss other methods such as a drug, placed in a hydrophilic medium, which is bonded on to the outside surface of a delivery device such as a balloon catheter or stent. Such placement of the drug can vary from just a physical mix a covalent bond to the hydro-gel itself.

Special devices have been designed in order to access percutaneously the pericardium and then to deliver pharmacologic agents in the pericardial cavity, which is in close contact with the sub-epicardial coronary arteries. None of the above methods or devices, known as pericardial injection, have achieved clinical acceptance.

Igo et al. in U.S. Patent 5,643,895, describes a method for injecting various liquid agents into the pericardium however such methods have very little chance of providing the correct amount of the agent into the target area to be effective, since they depend on diffusion in a large pericardial cavity where fluid is continuously produced and absorbed.
US 5,354,279 describes a drug injection device comprising a body (10) having a proximal and a distal end; at least one needle (26) mounted for movement with respect to said body for selective movement away from said body, said needles having at least one bend adjacent a distal end thereof aimed at maintaining the needles under a mechanical stress which presses them outwardly onto the outer shank (12).

Recently, the technology has been perfected to cover vascular stents (metallic endovascular prosthesis) with special coatings, able to carry and then deliver (small) doses of drugs. Cordis/Johnson & Johnson produce a stent featuring polymer coating carrying small quantities of sirolimus, an anti-proliferative drug. Specifically, such stents are been evaluated to treat or prevent restenosis in stents used for coronary angioplasty.
Several drugs and genetically engineered products (potentially, also virus-mediated nuclear transfusion of altered DNA material) are being tested for local delivery. The following intrinsic features potentially limit the coated/medicated stent technology: They need to be developed as a unit of stent/coating/drug, in a complex and expensive process; medicated stents provide limited amount of drugs;
medicated stents can only deliver the drugs over a limited time, possibly by chemical gradient migration, or diffusion at the external wall of the stent; the active principle may have variable migration capacity, depending on the arterial wall anatomic features (fibrotic capsule, cholesterol or calcium deposits) or cellular composition (especially, concentration of macrophages); and the necessary utilization of stents, to carry the active drugs. Such active drugs may be more effective (and economical) in the absence of a stent. In the case of in-stent restenosis (recurrent obstruction, inside a previously installed stent), the use of stent inside prior stents increases the probability of new recurrence of obstruction. Additionally, it is likely that many lesions commonly treated at the present time with a stent without drug coating, may have a better and more economical result by use of stent alone and balloon angioplasty when accompanied by effective anti-proliferative medication.

For all these reasons it is believed that a specially designed device, that can reliably inject, subintimally, adequate amounts of phramacologic agents, could improve the results of angioplasty, while limiting the cost of the intervention. It must be noted that, the absolute amount of scar tissue caused by stents is much larger than the scarring caused by stand-alone balloon angioplasty. It is the larger inner lumen that can be generally achieved with stents that can compensate for the increased scarring.

### SUMMARY OF THE INVENTION

The present invention is aimed at making a device that is quite simple to produce while effective for the intend purpose. Injecting the drug into the vessel wall is more effective than the transfer of the drug from the vessel lumen by means of diffusion. While "needle" methods of injecting the drug are conceptually desirable, the prior art devices have shortcomings that prevent them from achieving their desired purpose.

Additionally other body cavities or tubes may require similar technology for local pharmacological intervention (like the bile duct, bronchi, urethra, ureter, where both scar tissue or neoplastic growths could be addressed by such means).

The present invention uses several innovative means in order to effectively puncture the intimal wall of blood vessels reliably and controllably. The total penetration of the needle is controlled by the height of the needle projecting from its base and the degree of penetration is controlled by the use of "cam" or similar means or mechanisms that moves the base containing the needles.

This method has substantial advantages over the methods where the needle has been equipped with collars and natural spring actions or shape memory actions to make the penetration into the vessel wall. Springs are not reliable means to control the force required for penetration and the same is true for the force derived by shape memory means.

The present invention contains a distal segment attached to a proximal delivery catheter, where several sharp and thin needles are provided and kept in an enclosure, until they can be moved out controllably when actuated from the proximal end of the catheter, either by turning a knob or moving a cam type mechanism. Until the needles are moved out of the cage, the needles are safely harbored and cannot cause any damage to the vessels during advancing or retracting of the device. The design of the cage is such that the movement of the tip of the catheter in the vessel will not cause vascular damage. As such the cage is preferably oblong with smooth surfaces, which is advanced over a guide wire. Once the needles are projected from the device the penetration is limited by the amount the needle can project from its base.

The needle advancing mechanism can be spring loaded with stops to control the degree of projection, and has a feature allowing immediate retrieval when the needle is withdrawn. Sequentially, multiple injections in a target region can be accomplished by slowly withdrawing the catheter while alternatively pressing and releasing the knob responsible for injecting/retrieving the needles. By so doing, a vascular segment 2-3 cm long can be treated within 5-10 minutes or less. During this process not only the pharmacological agents in liquid form are released, but also pharmacological agents, which are prepackaged in micro-capsules, can be injected into the vessel wall or the underlying tissue to be released over time. In this case the drug can be either pre-mixed into a solid "ball" of biodegradable materials or encapsulated in a thin coat, which is absorbed into the tissue releasing the active drug.

The cage itself is a low profile structure that permits blood flow before and during the injection phase.

### DETAILED DESCRIPTION OF THE DRAWINGS

Figure 1 shows one embodiment with the needles retracted;

Figures 2A and 2B show an alternative embodiment as the needles are being extended;

Figure 3 is an alternative embodiment with the needles in a cage;

Figure 4 shows a moving wedge forcing out the needles;

Figures 5 and 5A are an alternative embodiment to Figure 4;

Figures 6A-6C show a sheath holding and releasing the needles;

Figures 7A-7D show an alternative embodiment where the tip aids in pushing out the needles; and

Figure 8 shows an alternative embodiment which is not comprised within the scope of the claims using vacuum to hold a housing to the tissue as pressure is used to inject drugs.

### DETAILED DESCRIPTION OF THE PREFERRED EMBODIMENT

The object of this invention is to develop a simple and effective device for injecting various anti-proliferative and other pharmacological agents into a target area of the body cavity in an even manner. The preferred embodiment comprises a delivery catheter, which is a slender tube or catheter typically 30-150cm long to reach different body cavities, with controlling means located at its proximal end and the drug delivery module located at its distal end. A lumen is provided to enable the catheter to be advanced over a guide wire whenever required, as is in normal catheterization procedures.

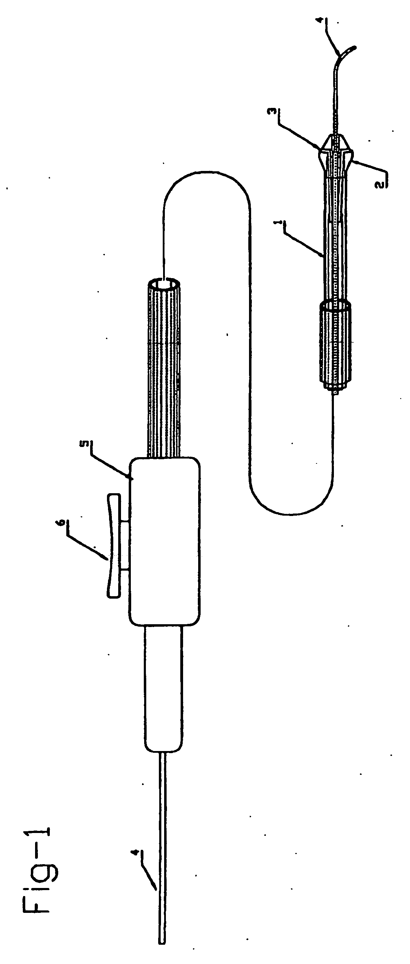

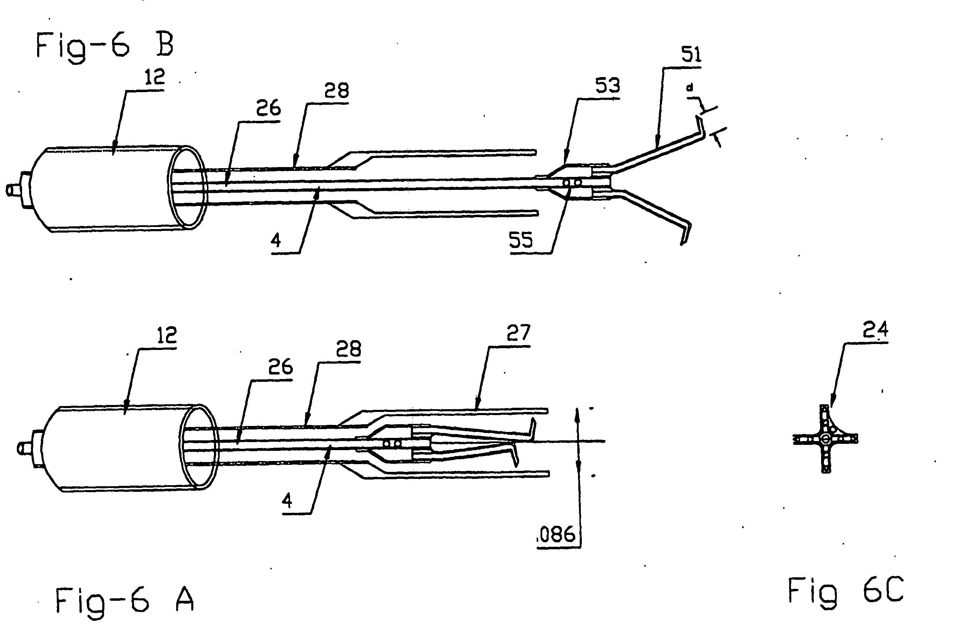

A typical embodiment of such is achieved by the catheter of Fig 1. This device has a distal end containing an injection head **2,** which carries a plurality of needles attached to at least one communicating channel that extends through the catheter body **1** to the proximal end **5** that contains the needle driving mechanism having a needle-driving control unit or knob **6**. This device can be advanced into the target area over a guide wire **4** for which a lumen has been provided therein. The injection head contains the retractable needles **3** that project out when activated but otherwise stay within the injection head. The injection head **2** can be spherical, elliptical or wing shaped as shown in Fig 6 depending on the application of the device depending on whether there is fluid flow (such as blood flow) in the lumen or cavity it is used in. Other shapes may be used.

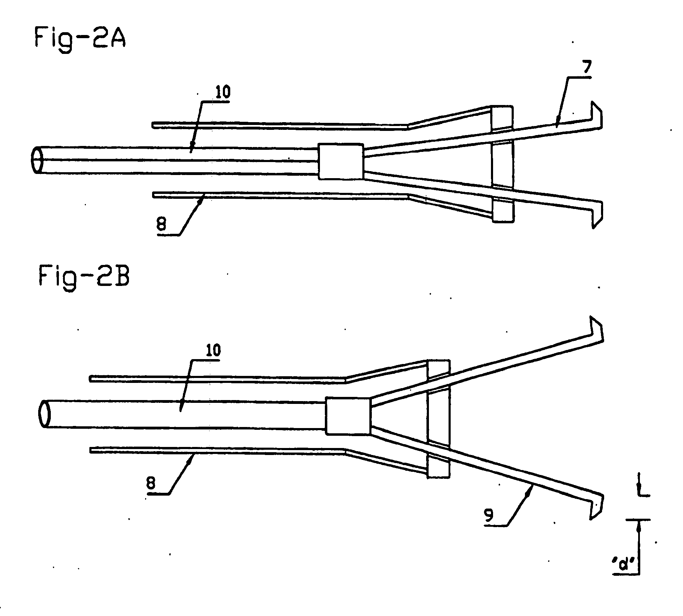

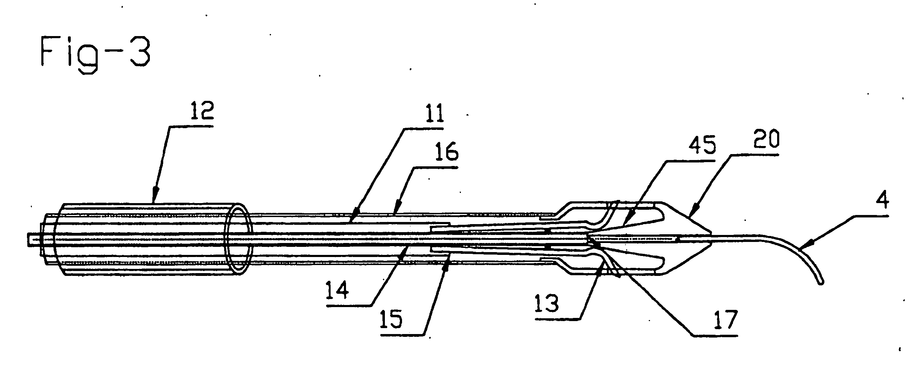

The general principle is shown in Fig 2. The needles **7** are designed with a nearly 90-degree angle at the tip (other angles can also be used depending on the design of the device and the application) and the height of the needle above the base of the needle is fixed (height "d") and controlled and predetermined. In order to have different degrees of penetration, in different cavities, it is safe to predetermine this height and use a device specific for the need. For example a small artery with a wall thickness of 2mm, the needle height "d" can be 1.5 mm while an artery with a wall thickness of 3mm can use a needle with needle height "d" of 2mm. As shown in Fig2 (B) the needles project outward when the outer casing or sheath is slid proximally. The geometrical fixation of the slide head **8** controllably moves the needles positively outwards without having to depend on spring action or shape memory action. When the slide head **8** is moved distally, the needles retract inwardly away from the wall of the artery and into the injection head **2**. The penetration is controlled by the height "d" of the needle protrusion, whereby when the shank **9** or the body of the needle rests against the vessel or luminal wall no further penetration is possible even when additional force is applied to the needle assembly. When not projecting, the needles are housed within the injection head **20** (see Figure 3) or are up against the slide head **8** in such a way not to cause any abrasion of the intimal wall during advancement or manipulation of the catheter.

The basic concept is to move the needles outward using a cam slider mechanism located in the handle at proximal end **5** and actuated by a knob **6**. Limiting the penetration of the tissue by the needle is accomplished by limiting the projected height of the needle above its base. Several alternative ways of achieving this goal will now be described.

Figure 3 describes one situation where the needles are safely housed within the injection head **20**. The device **11** in Fig 3 is advanced into target area through a guiding catheter **12** and over a guide wire **4**. The device **11** consists of an inner tube assembly to which the needles **13** are attached in fluid communication with an inner tube assembly **14** and **15**. When the outer casing **16** is moved backwards (proximally) the needles **13** will move out of the injection head and will be available to inject medications into the vessel wall. And when the outer casing **16** is moved distally the needles retract into the cage **20** completely enclosing the needles in the injection head. This is accomplished by actuating the cam **45** inside the injection head **20.**

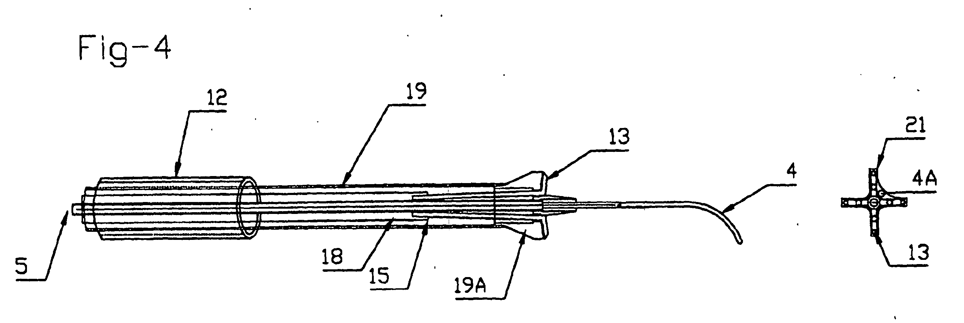

Alternatively, as shown in Figure 4, the needles **13** are attached to one tube **15** that extends along the length of the catheter to the proximal end **5** and to the drug source. The injection head **19A** is attached to tube **19** that continues along the catheter to the proximal end **6**. The through lumen **14** permits a guide wire **47** to be advanced through the catheter, enabling the positioning of the catheter over the guide wire **47** in the target vessel segment. A lumen 4A provided in the injection head **19A** will also permit the catheter to be advanced to the target area. This type of design will enable the device to be exchanged without having to remove the guide wire **47**, which is left in place to complete an angioplasty procedure (balloon and/or stent placement). The needles **13** in this design abut the injection head **19A** until it is moved proximally with respect to tube **15**. A spring loading mechanism (not shown) provided in the proximal end **5**, maintains the needles **13** in the retracted position at all times.

The needles **13** are connected to tube **15**, which is more or less central, and preferably metallic. The tube **19** is moved forward and backward by manipulating the knob **6** in the proximal end **5**, which causes the needles to expand and retract from the injection head.

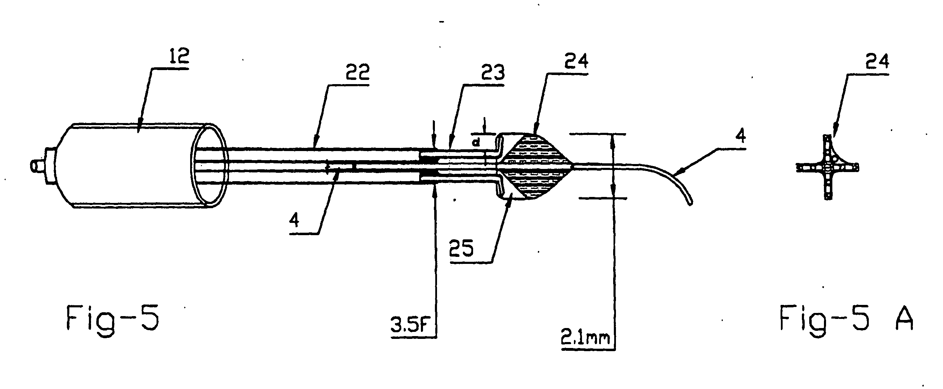

In yet another configuration, as shown in Figure 5, needles **49** move outward by using an olive-like member **24** attached to inner tube **30.** The pressure can be controlled by the degree to which the olive-like member **24** is retracted into the needles **49** and the depth of penetration is controlled by the height "d" of the needles **49** as has been described earlier. As shown in Figure 5, a plurality of needles **49** can be provided in order to inject the medications uniformly. When the needles **49** are not projected outwards, they are maintained within the housing **25**. The housing **25** may have different variations in its design; especially its distal end may be similar to the injections heads shown in Figure 3 and Figure 4.

In each case the movement is accomplished by means of either a tapered cam **20** (Fig 3) or the cam with "holes" as in **21**(Fig 4), which may or may not be a part of the cage. When the cam is moved proximally the needles project out and when the cam is moved distally the needles retract.

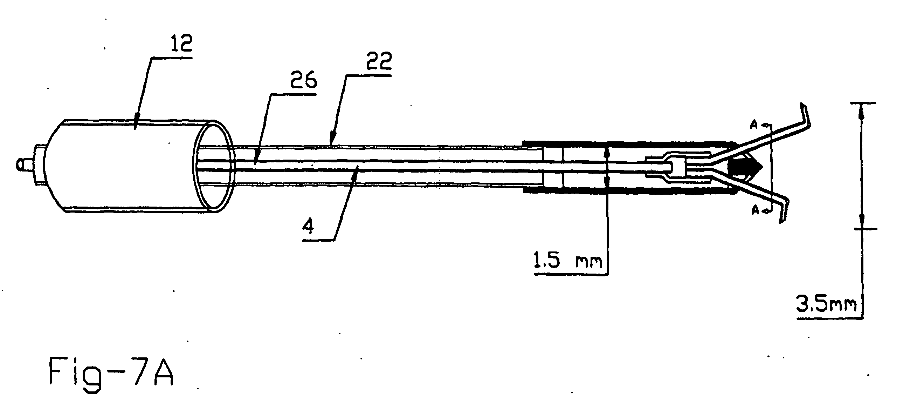

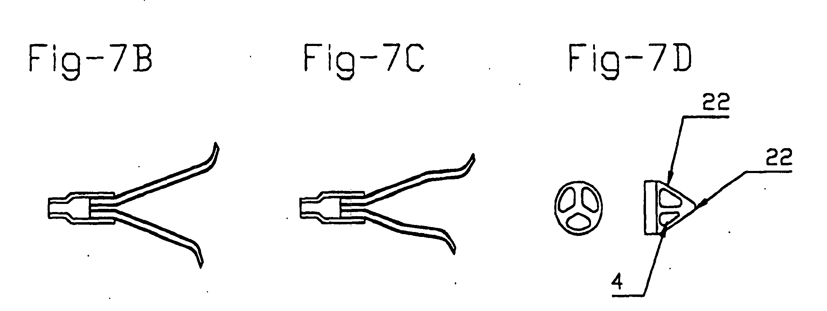

Still another variation of the invention is shown in Figure 6A and Figure 6B. In this design the needles **51** are attached to an inner tube **26**, which is in fluid communication with the injection head **53** and the injection needles **51**. The tube **26** is a flexible plastic tube made from such materials like nylon, polyethylene, polyurethane, polyimide etc. and may contain a spring coil or a stylet **55** at least for a portion of its length to provide resistance to kinking as well as to enhance pushability of this inner member. The outer sheath contains a housing **27**, made from metal or plastic to maintain the needle assembly enclosed during the advancement of the catheter to the arteries of the heart or other parts of the human body. An additional lumen **28** is provided on the outer or inner wall of the sheath **27**. The outer sheath **27** can also be made from a single piece. When the inner tube member **26** is pushed out or when the sheath **27** is pulled back the needles **51** will spring out as shown in Figure 6B. The needles **51** have an angular shape at their ends causing the needles depth of penetration to the height "d" (as discussed before in Figure 2B) of the needle above its base. The direction of the needle tip can be projecting perpendicular (Figure 6B) to the axis of the artery, projecting forward (Figure 7B) or projecting backwards (Fig 7A), depending on the actual action imposed on the device to incur penetration of the needles into the artery.

The distal end of the sheath may also contain a diverting mechanism as shown in Fig 7D. This structure generally has a bullet shaped end, **53** for ease of entry to tight lesions. The structure has a head, **55** divided into three (for accommodating three needles) As the sheath is moved back, the head will push against the needles to force them to positively deflect it outwards through the holes, **57** located in the head, **55** in addition to their normal deflection due to their spring action making a positive and controlled deflection. The diameter of the holes **57** are considerably larger than the needle diameter to minimize any friction which would be the case if the clearance between the needles and the hole is very small to help the penetration of the tissue where such additional force of penetration is required.

The needle assembly in Fig 6A shows the arrangement of the needles **51** which have different lengths to be staggered so that when the needles **51** are collapsed inside the cage each needle will not interfere with the other needle. This allows conserving space when the needles are folded into the sheath.

Figure 7C shows that the needles have an additional bend to provide a land area **59**, so that when the needle is deflected the land area **59** will help the needle to rest on the tissue preventing over penetration of the needle as previously discussed.

Depending on the size of the catheter and the location where drug therapy is needed, the number of needles can be 2 or more. Typically 2-6 needles are sufficient to inject a pharmacological agents uniformly into the vessel wall.

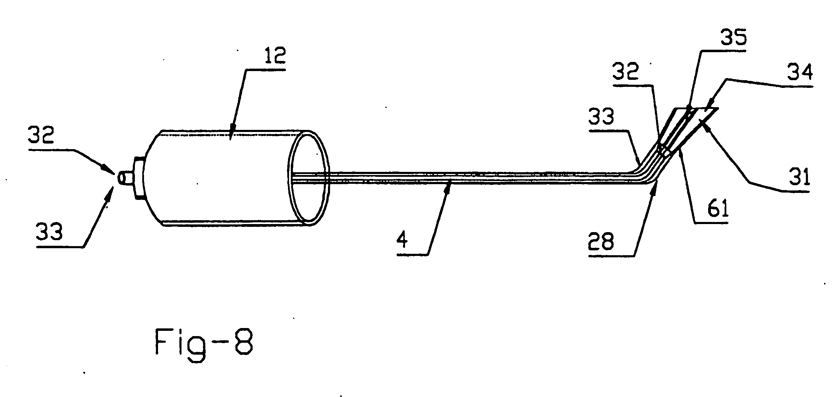

In another version which is not comprised within the scope of the claims of the present invention, drugs in its liquid form or encapsulated drugs can be implanted into the heart muscle or the walls of the coronary artery using the device shown in Fig 8. This device contains a small suction cup **31** made of metal or plastic with numerous holes **34**. The application of suction to a lumen connected to these suction holes will hold the cup firmly against the wall of the vessel of the heart. Once this is achieved the needle **35** can be advanced to a stop **61** to penetrate the wall and the drugs can be delivered either under pressure or the encapsulated solid form pharmaceuticals can be delivered using a push rod mechanism. The neck section **28** is bent and is made of flexible material so as to adjust to the required angle. The drugs are delivered by applying pressure to lumen **32** and the suction cup is held in place by applying suction to lumen **33**.

## Claims

1. A drug injection device for delivering at least one pharmacological agent in a body cavity, comprising:
a body (1) having a proximal (5) and distal (2) end;
at least one needle (3, 7) mounted for movement with respect to said body (1) for selective movement away from said body (1), said needle (3, 7) having at least one bend adjacent a distal end thereof and **characterized in that** said bend is located outside of said body when it limits penetration of said needle into a wall defining a body cavity.

2. The device of claim 1**,** wherein:
said at least one needle comprises a plurality of needles of different lengths.

3. The device of claim 2, wherein:
said needle is made from one of stainless steel and a shape memory alloy and is one of round and rectangular in cross-section.

4. The device of claim 1, wherein:
said bend defines an included angle that is one of acute, right, or obtuse.

5. The device of claim 1, wherein:
said needle is housed within said body for delivery to the cavity.

6. The device of claim 5, wherein:
said body comprises a sheath to cover said distal end of said needle for delivery to the body cavity, whereupon relative motion between said sheath and said needle, a stored force on said needle is released to drive said distal end up to said bend into the wall defining the body cavity.

7. The device of claim 1, wherein:
said at least one bend comprises at least a proximal and a distal bend, said distal end, up to said distal bend, penetrating the wall defining the body cavity and said second bend defining a segment of said needle between said bends that contacts the wall that defines the body cavity.

8. The device of claim 7, wherein:
said proximal bend biases said segment of said needle toward the wall that defines the body cavity.

9. The device of claim 1, wherein:
said body comprises a lumen to allow said body to be advanced over a guide wire and to be removed while leaving the guide wire in place.

10. The device of claim 9, further comprising:
one of a sheath and a guiding catheter through which said body can be guided to the body cavity.

11. The device of claim 1, wherein
said distal end of said needle is cammed away from said body.

12. The device of claim 11, wherein:
said body comprising a longitudinal axis and an opening oriented askew to said longitudinal axis, said needle extending through said opening, whereupon relative movement between said body and said needle, said distal end of said needle is selectively extended and retracted with respect to the wall defining the body cavity.

13. The device of claim 11, wherein:
said camming occurs by relative movement between a tapered surface of said body and said needle.

14. The device of claim 13, wherein:
said tapered surface contacts said needle adjacent said bend.

15. The device of claim 13, wherein:
said needle comprises a proximate end and said tapered surface contacts said needle adjacent said proximate end.

16. The device of claim 13, wherein:
camming of said distal end of said needle away from said body is accomplished by said tapered surface upon relative movement in a first direction, whereupon reversal of said relative movement, said distal segment retracts due to one of a stored force therein and shape memory.

## Patentansprüche

1. Eine Medikamenteninjektionsvorrichtung für die Freisetzung mindestens eines pharmakologischen Wirkstoffs in einer Körperhöhle, bestehend aus :
Einem Körper (1) mit einem proximalen (5) und einem distalen (2) Ende
Mindestens einer Nadel (3, 7), angebracht für eine Bewegung im Verhältnis zum genannten Körper (1) für die selektive Bewegung weg von diesem Körper (1), wobei diese Nadel (3, 7) mindestens eine Krümmung unmittelbar neben ihrem distalen Ende aufweist und welche sich außerhalb dieses Körpers befindet, wenn sie die Penetration dieser Nadel in eine Köperhöhle begrenzende Wand einschränkt.

2. Die Vorrichtung nach Anspruch 1, in welcher:
Diese mindestens eine Nadel eine Vielzahl von Nadeln unterschiedlicher Länge umfasst,

3. Die Vorrichtung nach Anspruch 2, in welcher:
Diese Nadel entweder aus Edelstahl oder aus einer Formgedächtnis-Legierung hergestellt und im Querschnitt entweder rund oder rechteckig ausgeführt ist,

4. Die Vorrichtung nach Anspruch 1, in welcher:
Diese Biegung einen eingeschlossenen Winkel definiert, der entweder ein spitzer, ein rechter oder ein stumpfer Winkel ist.

5. Die Vorrichtung nach Anspruch 1, in welcher:
Diese Nadel zur Zuführung in den Hohlraum im genannten Körper angeordnet ist.

6. Die Vorrichtung nach Anspruch 5, in welcher:
Der genannte Körper aus einer Hülle zur Abdeckung dieses distalen Endes dieser Nadel für die Zuführung in die Körperhöhle besteht, wo nach einer relativen Bewegung zwischen dieser Hülle und dieser Nadel eine gespeicherte Kraft auf jene Nadel freigesetzt wird, mit der das genannte distale Ende bis zur genannten Biegung in der Wand vorgetrieben wird, welche die Körperhöhle begrenzt.

7. Die Vorrichtung nach Anspruch 1, in welcher:
Diese, mindestens eine, Biegung aus mindestens einer proximalen und einer distalen Biegung besteht, wobei das genannte distale Ende bis zur genannten distalen Biegung in jene Wand eindringt, welche die Köperhöhle begrenzt, und wobei diese zweite Biegung ein Segment dieser Nadel zwischen den genannten Biegungen begrenzt, das mit der Wand in Berührung steht, welche die Körperhöhle begrenzt.

8. Die Vorrichtung nach Anspruch 7, in welcher:
Durch diese proximale Biegung das genannte Segment dieser Nadel in Richtung jener Wand neigt, welche die Körperhöhle begrenzt.

9. Die Vorrichtung nach Anspruch 1, in welcher:
Der genannte Körper aus einem Lumen besteht, welches den Vorschub dieses Körpers über einen Führungsdraht sowie die Rückholung dieses Körpers erlaubt, während der Führungsdraht an seinem Platz verbleibt.

10. Die Vorrichtung nach Anspruch 9, des Weiteren bestehend aus:
Entweder einer Hülle oder einem Führungskatheter, durch welche (n) dieser Körper zur Körperhöhle geführt werden kann.

11. Die Vorrichtung nach Anspruch 1, in welcher:
Das genannte distale Ende dieser Nadel vom genannten Körper weg versetzt wird.

12. Die Vorrichtung nach Anspruch 11, in welcher:
Der genannte Körper aus einer Längsachse und einer schräg an der genannten Längsachse ausgerichteten Öffnung besteht, wobei sich diese Nadel durch diese Öffnung erstreckt, und wo nach relativer Bewegung zwischen diesem Körper und genannter Nadel das genannte distale Ende dieser Nadel im Verhältnis zu der die Körperhöhle begrenzenden Wand selektiv ausgefahren und eingezogen wird.

13. Die Vorrichtung nach Anspruch 11, in welcher:
Der genannte Versatz durch die relative Bewegung zwischen einer konischen Oberfläche dieses Körpers und dieser Nadel eintritt.

14. Die Vorrichtung nach Anspruch 13, in welcher:
Diese konische Oberfläche diese Nadel neben dieser Biegung berührt.

15. Die Vorrichtung nach Anspruch 13, in welcher:
Diese Nadel aus einem proximalen Ende besteht und diese konische Oberfläche diese Nadel neben diesem proximalen Ende berührt.

16. Die Vorrichtung nach Anspruch 13, in welcher:
Der Versatz dieses distalen Endes dieser Nadel weg vom genannten Körper durch diese konische Oberfläche nach relativer Bewegung in eine ersten Richtung erreicht wird, bei der sich nach Umkehr dieser relativen Bewegung das genannte distale Segment aufgrund entweder der in ihm gespeicherten Kraft oder aufgrund des Formgedächtnisses zurückzieht.

## Revendications

1. Un dispositif d'administration de médicaments d'au moins un agent pharmacologique dans une cavité corporelle, comprenant :
un corps (1) ayant une extrémité proximale (5) et distale (2);
au moins une aiguille (3, 7) permettant un mouvement relatif audit corps (1) et un mouvement sélectif en dehors dudit corps (1), lesdites aiguilles (3, 7) ayant au moins une courbe adjacente à une extrémité distale et située en dehors dudit corps limitant la pénétration de ladite aiguille dans une paroi déterminant une cavité corporelle.

2. Dispositif suivant la revendication 1 :
**caractérisé en ce qu'**au moins une seringue comprend plusieurs aiguilles de différentes longueurs,

3. Dispositif suivant la revendication 2 :
**caractérisé en ce que** l'aiguille est faite d'acier inoxydable et d'alliage à mémoire de forme et est ronde et rectangle en coupe transversale,

4. Dispositif suivant la revendication 1 :
**caractérisé en ce que** la courbe a un angle d'ouverture qui est aigu, droit ou obtus.

5. Dispositif suivant la revendication 1 :
**caractérisé en ce que** l'aiguille est logée dans ledit corps pour son acheminement vers la cavité.

6. Dispositif suivant la revendication 5 :
**caractérisé en ce que** le corps comprend un fourreau pour couvrir ladite extrémité distale de ladite aiguille pour son acheminement vers la cavité corporelle, et par un mouvement relatif entre ledit fourreau et ladite aiguille, une force emmagasinée sur ladite aiguille est libérée pour écarter ladite extrémité distale de ladite courbure dans la paroi déterminant la cavité corporelle.

7. Dispositif suivant la revendication 1 :
**caractérisé en ce qu'**au moins une courbure comprend au moins une courbure proximale et distale, ladite extrémité distale, jusqu'à ladite courbure distale, pénétrant la paroi déterminant la cavité corporelle et ladite deuxième courbure déterminant un segment de ladite aiguille entre lesdites courbures qui touche la paroi déterminant la cavité corporelle.

8. Dispositif suivant la revendication 7 :
**caractérisé en ce que** la courbure proximale biaise ledit segment de ladite aiguille contre la paroi déterminant la cavité corporelle.

9. Dispositif suivant la revendication 1 :
**caractérisé en ce que** ledit corps comprend un lumen pour permettre audit corps d'avancer grâce à un fil-guide et d'être retiré laissant le fil-guide en place.

10. Dispositif suivant la revendication 9, comprenant encore :
un fourreau et un cathéter guide par lequel ledit corps peut être guidé jusqu'à la cavité corporelle.

11. Dispositif suivant la revendication 1 :
**caractérisé en ce que** l'extrémité distale de ladite aiguille est retirée dudit corps.

12. Dispositif suivant la revendication 11 :
**caractérisé en ce que** le corps comprenant un axe longitudinal et une ouverture oblique par rapport audit axe longitudinal, ladite aiguille sortant de ladite ouverture, avec un mouvement relatif entre ledit corps et ladite aiguille, ladite extrémité distale de ladite aiguille est sélectivement sortie puis rentrée par rapport à la paroi déterminant la cavité corporelle.

13. Dispositif suivant la revendication 11 :
**caractérisé en ce qu'**un coincement se produit par un mouvement relatif entre une surface effilée dudit corps et de ladite aiguille.

14. Dispositif suivant la revendication 13 :
**caractérisé en ce que** ladite surface effilée touche ladite courbure adjacente à ladite aiguille.

15. Dispositif suivant la revendication 13 :
**caractérisé en ce que** ladite aiguille comprend une extrémité proximale et ladite surface effilée touche ladite aiguille adjacente à ladite extrémité proximale.

16. Dispositif suivant la revendication 13 :
**caractérisé en ce que** le coincement de ladite extrémité distal de ladite aiguille est accompli par ladite surface effilée par un mouvement relatif dans une première direction, après inversion dudit mouvement relatif, ledit segment distal retourne à l'intérieur par l'action de la force emmagasinée et la mémoire de forme.
